(19) Europäisches Patentamt
European Patent Office
Office européen des brevets

(11) Veröffentlichungsnummer: **0 362 645 B1**

(12) **EUROPÄISCHE PATENTSCHRIFT**

(45) Veröffentlichungstag der Patentschrift: **08.06.94**

(51) Int. Cl.$^5$: **A61K 31/535**, A61K 31/505, C07D 475/08

(21) Anmeldenummer: **89117610.9**

(22) Anmeldetag: **23.09.89**

# (54) Verwendung von Pteridinen zur Verhinderung der primären und sekundären Resistenz bei der Chemotherapie und diese Verbindungen enthaltende Arzneimittel.

(30) Priorität: **01.10.88 DE 3833393**

(43) Veröffentlichungstag der Anmeldung:
**11.04.90 Patentblatt 90/15**

(45) Bekanntmachung des Hinweises auf die Patenterteilung:
**08.06.94 Patentblatt 94/23**

(84) Benannte Vertragsstaaten:
**AT BE CH DE ES FR GB GR IT LI LU NL SE**

(56) Entgegenhaltungen:
**DE-A- 1 620 498**
**DE-A- 1 620 571**
**DE-A- 1 795 515**
**DE-A- 1 795 516**
**DE-A- 1 921 308**
**GB-A- 1 194 548**
**US-A- 3 557 105**
**US-A- 3 574 206**
**US-A- 4 767 761**

**INTERNATIONAL JOURNAL OF CANCER, Band 43, Nr. 3, März 1989, Seiten 487-491,Alan R. Liss, Inc.; N. RAMU et al.: "Circumvention of adriamycin resistance by-dipyridamole analogues: A structure-activity relationship study"**

(73) Patentinhaber: **Dr. Karl Thomae GmbH**

**D-88397 Biberach(DE)**

(72) Erfinder: **Heckel, Armin, Dr. Dipl.-Chem.**
**Geschwister-Scholl-Strasse 71**
**D-7950 Biberach 1(DE)**
Erfinder: **Lebsanft, Jörg, Dr. Dipl.-Biol.**
**Guardinistrasse 37**
**D-5300 Bonn 3(DE)**
Erfinder: **Bamberger, Uwe, Dr.**
**Grünweiler 90**
**D-7955 Ochsenhausen(DE)**
Erfinder: **Ramu, Avner, Dr.**
**36 Hachalutz Street**
**Jerusalem 96222(IL)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Bei der Behandlung von neoplastischen Erkrankungen werden u. a. Naturprodukte, z. B. Vinca Alkaloide wie Vinblastin, Vincristin oder Vindesin, Epipodophyllotoxine wie Etoposid oder Teniposid und Antibiotica wie Dactinomycin, Daunorubicin, Doxorubicin, Bleomycin, Mithramycin oder Mitomycin (siehe Goodman and Gilman's, The Pharmacological Basis of Therapeutics, Macmillan Publishing Company, New York, 7. Auflage, Seiten 1240-1247 und 1277-1289 (1985)) eingesetzt. Bei der Chemotherapie mit den oben aufgeführten Substanzen ist häufig zu beobachten, daß die zu behandelnden Tumoren auf Grund einer primären Resistenz oder auf Grund einer sich wegen einer vorgegangenen Therapie gebildeten sekundären Resistenz auf die Therapie nicht ansprechen, oder, daß nach Remission der Tumoren therapieresistente Tumorzellen möglicherweise latent verbleiben. Diese resistente Tumorzellen führen in der Regel zu einem späteren Zeitpunkt zum Rezidiv.

Die Pteridine der nachstehend erwähnten allgemeinen Formel I sind teilweise literaturbekannt. So weisen die in der DE-A-1,620,498, DE-A-1,620,571 und in der US-A-3,574,206 beschriebenen Pteridine cardiovasculäre Eigenschaften auf, insbesondere koronardilatatierende Wirkungen.

Es wurde gefunden, daß durch die Applikation eines Pteridins der Formel

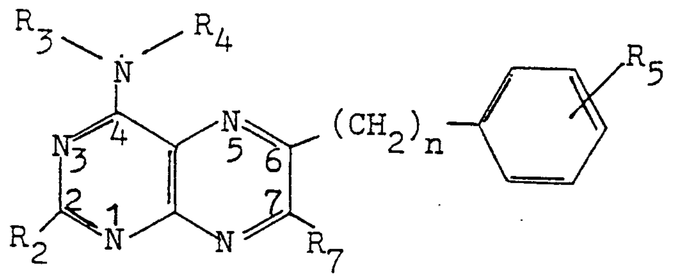

, (I)

in der

n die Zahl 0 oder 1,

$R_3$ eine geradkettige oder verzweigte Alkylgruppe mit 2 bis 6 Kohlenstoffatomen, in welcher mit Ausnahme des zum Stickstoffatom benachbarten Kohlenstoffatoms ein oder zwei Kohlenstoffatome durch eine Hydroxygruppe substituiert sind, und

$R_4$ eine Benzylgruppe oder eine Alkylgruppe mit 1 bis 4 Kohlenstoffatomen, in welcher mit Ausnahme des zum Stickstoffatom benachbarten Kohlenstoffatoms ein Kohlenstoffatom durch eine Hydroxygruppe substituiert sein kann, oder

$R_3$ eine Alkylgruppe mit 1 bis 3 Kohlenstoffatomen und

$R_4$ eine Phenylalkylgruppe mit 1 bis 3 Kohlenstoffatomen im Alkylteil,

$R_2$ und $R_7$, die gleich oder verschieden sein können, jeweils eine gegebenenfalls durch eine oder zwei Methyl- oder Ethylgruppen substituierte Pyrrolidino-, Piperidino- oder Morpholinogruppe, und

$R_5$ ein Wasserstoffatom oder eine Methylgruppe bedeuten, eines seiner geometrischen oder optischen Isomeren sowie dessen Säureadditionssalze, insbesondere dessen physiologisch verträglichen Säureadditionssalze mit einer anorganischen oder organischen Säure,

resistente Tumore zur Remission gebracht werden.

Durch die vorherige, gleichzeitige oder spätere Gabe eines Pteridins der obigen allgemeinen Formel I oder dessen physiologisch verträglichen Säureadditionssalzes wird erfindungsgemäß eine Sensibilisierung von Tumoren mit einer primären oder sekundären Resistenz gegenüber den oben erwähnten Chemotherapeutica erzielt.

Gegenstand der vorliegenden Erfindung sind daher neue Arzneimittel, enthaltend ein Pteridin der obigen allgemeinen Formel I, eines seiner geometrischen oder optischen Isomeren oder dessen physiologisch verträgliches Säureadditionssalz und mindestens ein Chemotherapeuticum zur Behandlung neoplastischer Erkrankungen aus der Reihe der Naturprodukte, und die neue Verwendung der obigen Pteridine zur Sensibilisierung von Tumoren bei der Chemotherapie mit den vorstehend erwähnten Chemotherapeutica aus der Reihe der Naturprodukte.

Für die bei der Definition der Reste eingangs erwähnten Bedeutungen kommt beispielsweise für

$R_2$ und $R_7$ jeweils die der Pyrrolidino-, 2-Methyl-pyrrolidino-, 3-Methyl-pyrrolidino-, 3,3-Dimethyl-pyrrolidino-, 2-Ethyl-pyrrolidino-, 3-Ethyl-pyrrolidino-, 3,3-Diethyl-pyrrolidino-,Piperidino-, 2-Methyl-piperidino-, 3-Methyl-piperidino-, 4-Methyl-piperidino-, 2-Ethyl-piperidino-, 3-Ethyl-piperidino-, 4-Ethyl-piperidino-, 3,5-Dimethyl-piperidino-, 3,5-Diethyl-piperidino-, Morpholino-, 2-Methyl-morpholino-, 2-Ethyl-morpholino-, 3-Methyl-morpholino-, 3-Ethyl-morpholino-, 2,6-Dimethyl-morpholino-, cis-2,6-Dimethyl-morpholino-, trans-2,6-Dimethyl-morpholino-, 2,6-Diethyl-morpholino-, cis-2,6-Diethyl-morpholino-, trans-2,6-Diethyl-morpholino-, 3,5-Dimethyl-morpholino- oder 3,5-Diethyl-morpholinogruppe,
für $R_3$ die der 2-Hydroxy-ethyl-, 2-Hydroxy-n-propyl-, 2-Hydroxy-isopropyl-, 3-Hydroxy-propyl-, 4-Hydroxy-n-butyl-, 2-Hydroxy-2-methyl-n-propyl-, 5-Hydroxy-n-pentyl-, 6-Hydroxy-n-hexyl-, 2,3-Dihydroxy-n-propyl-, Methyl-, Ethyl-, n-Propyl- oder Isopropylgruppe,
für $R_4$ die der Benzyl-, 2-Phenyl-ethyl-, 2-Phenyl-n-propyl-, 3-Phenyl-propyl-, 2-Hydroxy-ethyl-, 2-Hydroxy-n-propyl-, 3-Hydroxy-propyl-, 4-Hydroxy-n-butyl- oder 2-Hydroxy-2-methyl-n-propylgruppe und
für $R_5$ die des Wasserstoffatoms oder der Methylgruppe in Betracht.

Bevorzugte Pteridine der obigen allgemeinen Formel I sind hierbei diejenigen, in denen
n die Zahl 0,
$R_2$ und $R_7$, die gleich oder verschieden sein können, eine Piperidino-, 3,5-Dimethyl-piperidino-, Morpholino-, 2-Methyl-morpholino-, 2,6-Dimethyl-morpholino-, cis-2,6-Dimethyl-morpholino- oder trans-2,6-Dimethyl-morpholinogruppe,
$R_3$ eine Methyl-, Ethyl-, Benzyl-, 2-Hydroxy-ethyl-, 2-Hydroxy-n-propyl-, 2,3-Dihydroxy-n-propyl- oder 2-Methyl-2-hydroxy-n-propylgruppe,
$R_4$ eine 2-Hydroxy-ethyl-, 2-Hydroxy-n-propyl- oder 2-Methyl-2-hydroxy-n-propylgruppe und
$R_5$ ein Wasserstoffatom oder eine Methylgruppe bedeuten,
deren optische und geometrische Isomere sowie deren physiologisch verträgliche Säureadditionssalze, wobei die Verbindungen
4-[N-(2-Hydroxy-2-methyl-n-propyl)-ethanolamino]-2,7-bis(2,6-dimethyl-morpholino)-6-phenyl-pteridin,
4-(N-Ethyl-ethanolamino)-2,7-bis(2,6-dimethyl-morpholino)-6-phenyl-pteridin,
4-(Ethanol-isopropanolamino)-2,7-bis(2,6-dimethyl-morpholino)-6-phenyl-pteridin,
4-[N-(2-Hydroxy-2-methyl-n-propyl)-ethanolamino]-2,7-bis(2-methyl-morpholino)-6-phenyl-pteridin,
4-(Ethanol-isopropanolamino)-2,7-bis(2-methyl-morpholino)-6-benzyl-pteridin,
4-(Ethanol-isopropanolamino)-2,7-bis(2-methyl-morpholino)-6-(o-tolyl)-pteridin,
4-[Bis(2-hydroxy-2-methyl-n-propyl)-amino]-2,7-bis(2,6-dimethyl-morpholino)-6-phenyl-pteridin,
4-(2,3-Dihydroxy-n-propyl-ethanolamino)-2,7-bis(2,6-dimethylmorpholino)-6-phenyl-pteridin,
4-[N-(2-Hydroxy-2-methyl-n-propyl)-ethanolamino]-2,7-bis-(3,5-dimethyl-piperidino)-6-phenyl-pteridin,
4-(N-Ethanol-benzylamino)-2,7-bis(2,6-dimethyl-morpholino)-6-phenyl-pteridin,
4-[N-(2-Hydroxy-2-methyl-n-propyl)-ethanolamino]-2,7-bis(cis-2,6-dimethyl-morpholino)-6-phenyl-pteridin,
4-[N-(2-Hydroxy-2-methyl-n-propyl)-ethanolamino]-2-(2,6-dimethyl-morpholino)-7-morpholino-6-phenyl-pteridin,
4-[N-(2-Hydroxy-2-methyl-n-propyl)-ethanolamino]-7-(2,6-dimethyl-morpholino)-2-morpholino-6-phenyl-pteridin und
4-(N-Ethyl-benzylamino)-2,7-bis(2,6-dimethyl-morpholino)-6-phenyl-pteridin
neu sind.

Die vorstehend erwähnten neuen Pteridine, Verfahren zu ihrer Herstellung und diese Pteridine enthaltende Arzneimittel sind ein weiterer Gegenstand der vorliegenden Erfindung.

Bevorzugte neue Verbindungen sind jedoch diejenigen, in denen
n die Zahl 0,
$R_2$ und $R_7$ jeweils eine 2,6-Dimethyl-morpholinogruppe oder
einer der Reste $R_2$ oder $R_7$ eine Morpholinogruppe und
der andere der Reste $R_2$ oder $R_7$ eine 2,6-Dimethyl-morpholinogruppe,
$R_3$ und $R_4$ zusammen eine N-(2-Hydroxy-2-methyl-n-propyl)-ethanolaminogruppe und
$R_5$ ein Wasserstoffatom bedeuten.

Erfindungsgemäß haben sich folgende Pteridine als besonders wirksam erwiesen:
4-[N-(2-Hydroxy-2-methyl-n-propyl)-ethanolamino]-2,7-bis(2,6-dimethyl-morpholino)-6-phenyl-pteridin,
4-(N-Ethyl-ethanolamino)-2,7-bis(2,6-dimethyl-morpholino)-6-phenyl-pteridin,
4-(Ethanol-isopropanolamino)-2,7-bis(2,6-dimethyl-morpholino)-6-phenyl-pteridin,
4-(Diethanolamino)-2,7-bis(2,6-dimethyl-morpholino)-6-phenyl-pteridin,
4-[N-(2-Hydroxy-2-methyl-n-propyl)-ethanolamino]-2,7-bis(2-methyl-morpholino)-6-phenyl-pteridin,
4-(Ethanol-isopropanolamino)-2,7-bis(2-methyl-morpholino)-6-benzyl-pteridin,
4-(Ethanol-isopropanolamino)-2,7-bis(2-methyl-morpholino)-6-phenyl-pteridin,
4-(Etnanol-isopropanolamino)-2,7-bis(2-methyl-morpholino)-6-(o-tolyl)-pteridin,
4-(N-Benzyl-ethanolamino)-2,7-dimorpholino-6-phenyl-pteridin,
4-[Bis(2-hydroxy-2-methyl-n-propyl)-aminol-2,7-bis(2,6-dimethyl-morpholino)-6-phenyl-pteridin,

4-(2,3-Dihydroxy-n-propyl-ethanolamino)-2,7-bis(2,6-dimethyl-morpholino)-6-phenyl-pteridin,
4-(Diethanolamino)-2,7-dipiperidino-6-phenyl-pteridin,
4-(Diethanolamino)-2,7-dimorpholino-6-phenyl-pteridin,
4-(N-Ethyl-ethanolamino)-2,7-dimorpholino-6-phenyl-pteridin,
4-(N-Methyl-ethanolamino)-2,7-dimorpholino-6-phenyl-pteridin,
4-[N-(2-Hydroxy-2-methyl-n-propyl)-ethanolamino]-2,7-bis-(3,5-dimethyl-piperidino)-6-phenyl-pteridin,
4-(N-Ethanol-benzylamino)-2,7-bis(2,6-dimethyl-morpholino)-6-phenyl-pteridin,
4-[N-(2-Hydroxy-2-methyl-n-propyl)-ethanolamino]-2,7-bis(cis-2,6-dimethyl-morpholino)-6-phenyl-pteridin,
4-[N-(2-Hydroxy-2-methyl-n-propyl)-ethanolamino]-2,7-bis-(trans-2,6-dimethyl-morpholino)-6-phenyl-pteridin,
4-[N-(2-Hydroxy-2-methyl-n-propyl)-ethanolamino]-2-(2,6-dimethyl-morpholino)-7-morpholino-6-phenyl-pteridin und
4-[N-(2-Hydroxy-2-methyl-n-propyl)-ethanolamino]-7-(2,6-dimethyl-morpholino)-2-morpholino-6-phenyl-pteridin,
sowie deren physiologisch verträgliche Säureadditionssalze.

Beispielsweise wurde die Sensibilisierung durch die Verbindungen

A = 4-[N-(2-Hydroxy-2-methyl-propyl)-ethanolamino]-2,7-bis-(2,6-dimethyl-morpholino)-6-phenyl-pteridin,
B = 4-Ethyl-ethanolamino-2,7-bis(2,6-dimethyl-morpholino)-6-phenyl-pteridin,
C = 4-Ethanol-isopropanolamino-2,7-bis(2,6-dimethyl-morpholino)-6-phenyl-pteridin,
D = 4-Diethanolamino-2,7-bis(2,6-dimethyl-morpholino)-6-phenyl-pteridin und
E = 4-[N-(2-Hydroxy-2-methyl-propyl)-ethanolamino]-2,7-bis(cis-2,6-dimethyl-morpholino)-6-phenyl-pteridin

am Beispiel von gegen Adriamycin resistenten Zellen wie folgt geprüft:
Proliferierende, adriamycinresistente S 180 Maus Sarcomazellen werden in Anwesenheit unterschiedlicher Konzentrationen Testsubstanzen für sechs Tage kultiviert. Zytotoxisch oder zytostatisch wirkende Konzentrationen der Testsubstanzen werden durch vermindertes Zellwachstum oder durch das Absterben der Zellen angezeigt. Endpunkt des Assays ist die Zahl der lebenden Zellen pro Kultur, die indirekt ermittelt wird, indem die Eigenschaft vitaler Zellen, den Farbstoff MTT [= 3-(4,5-Dimethylthiazol-2-yl)-2,5-diphenyltetrazoliumbromid] zum farbigen Formazan zu reduzieren, ausgenützt wird. Als IC50 wird die Konzentration einer Testsubstanz bezeichnet, welche die Zahl der vitalen Zellen pro Kulturgefäß auf 50 % der unbehandelten Kontrolle reduziert. Die Testsubstanzen werden sowohl in Abwesenheit von Adriamycin als auch in Anwesenheit einer unter Kulturbedingungen nicht proliferationshemmend wirkenden Menge Adriamycin getestet. Pro Testsubstanz erhält man daher zwei IC50-Werte, einen in Anwesenheit (IC50 ADR), den anderen in Abwesenheit (IC50) von Adriamycin. Die Differenz der Zehnerlogarithmen der beiden IC50-Werte: Δ = 1gIC50-1gIC50 ADR ist ein Maß für die Erhöhung der Zytotoxizität der Testsubstanz durch Adriamycin.

<u>Versuchungsdurchführung</u>:

Exponentiell wachsende, adriamycinresistente oder adriamycinsensitive S 180-Zellen werden in 96-Loch Flachboden-Mikrotiterplatten zu 2000 Zellen pro Loch in 100 µl Wachstumsmedium (RPMI-1640, das 10 % fötales Rinderserum enthält) ausplattiert. Die Kulturplatten werden im Brutschrank bei 37 °C, 5 % $CO_2$ und 100 % relativer Luftfeuchtigkeit inkubiert. Nach 24 Stunden werden pro Loch 50 µl Wachstumsmedium, das unterschiedliche Konzentrationen an Testsubstanz enthält, und 50 µl Wachstumsmedium mit oder ohne Adriamycin hinzugefügt. Im Anschluß an eine weitere sechstägige Kultivierung werden 50 µl Tetrazoliumsalzlösung [5 mg 3-(4,5-Dimethylthiazol-2-yl)-2,5-diphenyltetrazoliumbromid pro ml Phosphat-gepufferter Salinelösung, vor Gebrauch 1:5 (v/v) mit RPMI-1640 verdünnt] in jedes Loch pipettiert. Nach vier Stunden Inkubation wird das Kulturmedium vorsichtig abgesaugt und intrazellulär gebildetes Formazan durch 150 µl Dimethylsulfoxid pro Loch solubilisiert. Die Platten werden kurz geschüttelt und die optische Dichte bei 570 nm mit einem Photometer wie einem Dynatech MR-600-Gerät gemessen. Die Bildung des farbigen Formazans durch Reduktion des Tetrazoliumsalzes ist proportional der Zahl der lebenden Zellen. Die Mittelwerte von Dreifach-Bestimmungen wurden bei der Berechnung der IC50-Werte verwendet (Verdünnungsstufe: 1:2).

Tabelle 1

| Substanz | IC50 [µg/ml] Adriamycin in ng/ml 0 | 25 | lg IC50 / IC50 25 ng Adriamycin |
|---|---|---|---|
| A | 20,69 | 1,49 | 1,14 |
| B | > 31,60 | 4,43 | > 0,85 |
| C | 12,85 | 3,77 | 0,53 |
| D | 14,37 | 5,78 | 0,40 |
| E | 7,50 | 0,50 | 1,18 |

Tabelle 2

| Substanz | IC50 [µg/ml] Adriamycin in ng/ml 0 | 100 | lg IC50 / IC50 100 ng Adriamycin |
|---|---|---|---|
| A | 20,69 | < 0,50 | > 1,61 |
| B | > 31,60 | 0,77 | > 1,61 |
| C | 12,85 | 0,82 | 1,20 |
| D | 14,37 | 1,57 | 0,96 |
| E | 7,50 | 0,10 | 1,88 |

Die Pteridine der obigen allgemeinen Formel I weisen somit eine ausgeprägte Sensibilisierung auf adriamycinresistente Sarcomazellen auf und eignen sich daher in Kombination mit Vinca Alkaloiden, Epipodophyllotoxinen oder Antibiotica wie Daunorubicin, Doxorubicin, Bleomycin, Mithramycin oder Mitomycin zur Behandlung von neoplastischen Erkrankungen, um deren Resistenz gegenüber einer diesbezüglichen Chemotherapie aufzuheben und somit die Remission der gegenüber diesen Substanzen resistenten Tumoren zu bewirken. Bei gegenüber den erwähnten Chemotherapeutica sensiblen Tumoren verhindern die Pteridine der Formel I also zusammen mit dem Chemotherapeuticum, daß therapieresistente Tumorzellsubpopulationen die Therapie überleben und zum Rezidiv führen können.

Die Application der Pteridine, die überdies gut verträglich sind, erfolgt getrennt oder kombiniert mit einem in der üblichen Dosis applizierten Chemotherapeuticum; die Dosis des eingesetzten Pteridins liegt hierbei zwischen 1 und 50 mg/kg Körpergewicht pro Tag, vorzugsweise zwischen 3 bis 20 mg/kg Körpergewicht pro Tag, verteilt auf 1 bis 4 Einzeldosen.

Kombiniert mit einem entsprechenden Chemotherapeuticum kommt hierbei eine intravenöse Darreichungsform wie Ampullen und bei einer getrennten Application eine parallele Darreichungsform von Tabletten, Dragees, Suspensionen, Säfte, Kapseln oder Zäpfchen in Betracht.

Auf Grund des literaturbekannten Applicationsschemas der bei der Chemotherapie von neoplastischen Erkrankungen eingesetzten Naturprodukte (siehe Goodman and Gilman's, The Pharmacoligical Basis of Therapeutics, Macmillan Publishing Company, New York, 7. Auflage, Seiten 1240-1247 und 1277-1289 (1985)) erfolgt die erste Application eine Pteridins der Formel I oder dessen physiologisch verträglichem Säureadditionssalz zweckmäßigerweise zusammen mit oder vor dem verwendeten Chemotherapeuticum bzw. mit oder vor einer Kombination mehrerer Chemotherapeutica, die mindestens eines der vorstehend erwähnten Chemotherapeutica enthält (siehe DeVita et al. in "Cancer, Principles & Practice of Oncology", 2nd Edition, J. B. Lippincott Company Philadelphia).

Die übrigen Applikationen eines Pteridins der Formel I oder dessen physiologisch verträglichen Säureadditionssalzes können den Umständen entsprechend peroral oder ebenfalls intravenös erfolgen.

Eine erfindungsgemäß für die i.v.-Application geeignete Kombination enthält somit zweckmäßigerweise 1 bis 25 mg/kg, vorzugsweise 1 bis 20 mg/kg Körpergewicht, eines Pteridins der Formel I oder dessen physiologisch verträgliches Säureadditionssalz und ein geeignetes Chemotherapeuticum oder eine Kombination verschiedener geeigneter Chemotherapeutica, z. B.

0.1 - 0.15 mg/kg Vinblastin alle 7 Tage, wobei die Dosis abhängig vom Grad der Nebenwirkungen in Stufen von 0,05 mg/kg erhöht werden kann,
ca. 2 $mg/m^2$ Körperoberfläche Vincristin alle 7 Tage bei juveniler Leukämie, wobei bei Erwachsenen die Therapie vorzugsweise mit 0.01 mg/kg alle 7 Tage beginnt und je nach der Verträglichkeit bis auf 0.02 - 0.05 mg/kg gesteigert werden kann,
3 - 4 $mg/m^2$ Körperfläche Vindesin alle 7 Tage,
25 - 30 $\mu g/kg$ Mithramycin täglich oder alle 2 Tage,
60 - 75 $mg/m^2$ Körperoberfläche Adriamycin alle 21 Tage,
30 - 60 $mg/m^2$ Körperoberfläche Daunorubicin täglich für 3 Tage,
10 - 15 $\mu g/kg$ Dactinomycin täglich für 5 Tage,
50 - 100 $mg/m^2$ Körperoberfläche Etoposid täglich für 5 Tage und
30 $mg/m^2$ Körperoberfläche Teniposid täglich bis 5 Tage,
wobei jeweils nach einer 10-tägigen Pause sich 6 - 10 Behandlungszyklen anschließen.

Die Pteridine der allgemeinen Formel I werden in der CA-A-912.556, US-A-3.557.105 und US-A-3.574.206 beschrieben oder können nach den dort beschriebenen Verfahren hergestellt werden.

Die neuen Pteridine der obigen Formel I erhält man durch Umsetzung eines Pteridins der Formel

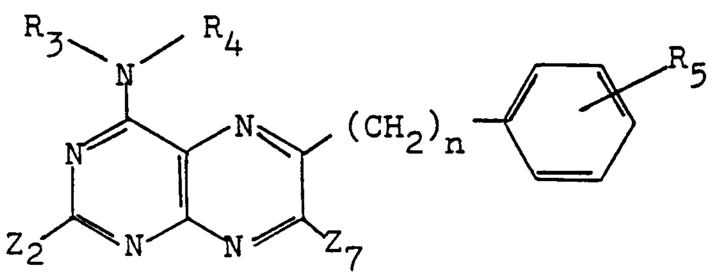

, (II)

in der
$R_3$ bis $R_5$ und n wie eingangs definiert sind,
einer der Reste $Z_2$ oder $Z_7$ eine nukleophil austauschbare Gruppe wie ein Halogenatom, z. B. ein Chlor- oder Bromatom, darstellt und
der andere der Reste $Z_2$ oder $Z_7$ die für $R_2$ oder $R_7$ eingangs erwähnten Bedeutungen besitzt oder ebenfalls eine nukleophil austauschbare Guppe wie ein Halogenatom, z. B. ein Chlor- oder Bromatom, darstellt, mit einem Amin der allgemeinen Formel

H - X ,(III)

in der
X die für $R_2$ oder $R_7$ eingangs erwähnten Bedeutungen besitzt.

Die Umsetzung wird zweckmäßigerweise in einem Lösungsmittel wie Tetrahydrofuran, Dioxan, Benzol, Toluol, Dimethylsulfoxid oder Dimethylglycoläther bei Temperaturen zwischen 0 und 150 °C, vorzugsweise

bei Temperaturen zwischen der Raumtemperatur und der Siedetemperatur des verwendeten Lösungsmittels oder in der Schmelze durchgeführt. Hierbei kann die Verwendung eines säurebindenden Mittels wie Natriumcarbonat, Triäthylamin oder Pyridin von Vorteil sein.

Die erfindungsgemäß erhaltenen neuen Verbindungen lassen sich in ihre Säureadditionssalze, insbesondere in ihre physiologisch verträglichen Salze mit anorganischen oder organischen Säuren überführen. Als Säuren kommen beispielsweise Salzsäure, Bromwasserstroffsäure, Schwefelsäure, Phosphorsäure, Milchsäure, Zitronensäure, Weinsäure, Bernsteinsäure, Maleinsäure oder Fumarsäure in Betracht.

Die als Ausgangsstoff verwendeten Verbindungen der allgemeinen Formel II und III sind zum größten Teil bekannt (siehe CA-A-912.556, US-A-3.557.105 und US-A-3.574.206) bzw. man erhält diese nach den in der US-A-2.940.972 oder gemäß den in den vorstehend erwähnten Patentschriften beschriebenen Verfahren.

Enthalten die Verbindungen der Formel I mindestens ein chirales Zentrum, so lassen sich diese mittels üblichen Methoden in ihre Enantiomeren auftrennen, beispielsweise durch Säulenchromatographie an einer chiralen Phase oder durch Kristallisation mit optisch aktiven Säuren, z.B. mit D- oder L-Monomethylweinsäure, D- oder L-Diacetyl-weinsäure, D- oder L-Weinsäure, D- oder L-Milchsäure oder D- oder L-Camphersäure.

Außerdem können die Verbindungen der Formel I, sofern diese mindestens ein geometrisches Zentrum wie eine durch zwei Methyl- oder Ethylgruppen substituierte Pyrrolidino-, Piperidino- oder Morpholinogruppe enthalten, insbesondere jedoch mindestens eine 2,6-Dimethyl-morpholinogruppe, mittels üblichen Methoden, beispielsweise durch Chromatographie, in ihre cis-/trans-Isomere aufgetrennt werden oder vorliegen.

Die nachfolgenden Beispiele sollen die Erfindung näher erläutern:

<u>Beispiel 1</u>

<u>4-[N-(2-Hydroxy-2-methyl-n-propyl)-ethanolamino]-2,7-bis(2,6-dimethyl-morpholino)-6-phenyl-pteridin</u>

a) 2,7-Dichlor-4-[N-(2-hydroxy-2-methyl-n-propyl)-ethanolamino]-6-phenyl-pteridin

15,5 g (0,005 Mol) 2,4,7-Trichlor-6-phenyl-pteridin werden in 100 ml Aceton gelöst und mit 13,3 g (0,1 Mol) N-(2-Hydroxy-2-methyl-n-propyl)-ethanolamin versetzt. Nach 20-minütigem Rühren bei Raumtemperatur wird die entstehende Suspension mit 200 ml Wasser versetzt. Das ausgefallene Produkt wird abgesaugt, mit Wasser gewaschen und aus Methanol/Wasser = 95 : 5 kristallisiert.
Ausbeute: 19,9 g (97 % der Theorie)
Schmelzpunkt: 145 - 147 °C.

b) 4-[N-(2-Hydroxy-2-methyl-n-propyl)-ethanolamino]-2,7-bis(2,6-dimethyl-morpholino)-6-phenyl-pteridin

2,0 g (0,005 Mol) 2,7-Dichlor-4-[N-(2-hydroxy-2-methyl-n-propyl)-ethanolamino]-6-phenyl-pteridin werden in 20 ml Dioxan gelöst und mit 3,5 g (0,08 Mol) 2,6-Dimethyl-morpholin 30 Minuten lang am Rückfluß erhitzt. Anschließend wird die Reaktionslösung in Wasser gegossen, der ausgefallene Niederschlag abgesaugt, mit Wasser gewaschen und aus Essigsäureethylester umkristallisiert.
Ausbeute: 2,4 g (84 % der Theorie),
Schmelzpunkt: 191 - 194 °C.

<u>Beispiel 2</u>

<u>4-(N-Ethyl-ethanolamino)-2,7-bis(2,6-dimethyl-morpholino)-6-phenyl-pteridin</u>

Hergestellt aus 4-(N-Ethyl-ethanolamino)-2,7-dichlor-6-phenyl-pteridin und 2,6-Dimethyl-morpholin analog Beispiel 1.
Ausbeute: 81 % der Theorie,
Schmelzpunkt: 128 - 138 °C (Ethanol).

Beispiel 3

4-(Ethanol-isopropanolamino)-2,7-bis(2,6-dimethyl-morpholino)-6-phenyl-pteridin

Hergestellt aus 4-(Ethanol-isopropanolamino))-2,7-dichlor-6-phenyl-pteridin und 2,6-Dimethyl-morpholin analog Beispiel 1.
Ausbeute: 60 % der Theorie,
Schmelzpunkt: 192 - 193 ° C (Methanol/Wasser = 4 : 1).

Beispiel 4

4-[N-(2-Hydroxy-2-methyl-n-propyl)-ethanolamino]-2,7-bis(2-methyl-morpholino)-6-phenyl-pteridin

Hergestellt aus 4-[N-(2-Hydroxy-2-methyl-n-propyl)-ethanolamino]-2,7-dichlor-6-phenyl-pteridin und 2-Methyl-morpholin analog Beispiel 1.
Ausbeute: 74 % der Theorie,
Schmelzpunkt: 143 - 147 ° C (Essigester/Petrolether).

Beispiel 5

4-(Ethanol-isopropanolamino)-2,7-bis(2-methyl-morpholino)-6-benzyl-pteridin

Hergestellt aus 4-(Ethanol-isopropanolamino)-2,7-dichlor-6-benzyl-pteridin und 2-Methyl-morpholin analog Beispiel 1.
Ausbeute: 55 % der Theorie,
Schmelzpunkt: 100 - 105 ° C.

Beispiel 6

4-(Ethanol-isopropanolamino)-2,7-bis(2-methyl-morpholino)-6-(o-tolyl)-pteridin

Hergestellt aus 4-(Ethanol-isopropanolamino)-2,7-dichlor-6-(o-tolyl)-pteridin und 2-Methyl-morpholin analog Beispiel 1.
Ausbeute: 85 % der Theorie,
Schmelzpunkt: 105 - 110 ° C (Umfällung aus 0,1 N Salzsäure).

Beispiel 7

4-[Bis(2-hydroxy-2-methyl-n-propyl)-amino]-2,7-bis(2,6-dimethyl-morpholino)-6-phenyl-pteridin

Hergestellt aus 4-[Bis(2-hydroxy-2-methyl-n-propyl)-amino]-2,7-dichlor-6-phenyl-pteridin und 2,6-Dimethyl-morpholin analog Beispiel 1.
Ausbeute: 46 % der Theorie,
Schmelzpunkt: 122 - 127 ° C (Umfällung aus 0,1 N Salzsäure).

Beispiel 8

4-(2,3-Dihydroxy-n-propyl-ethanolamino)-2,7-bis(2,6-dimethyl-morpholino)-6-phenyl-pteridin

Hergestellt aus 4-(2,3-Dihydroxy-n-propyl-ethanolamino)-2,7-dichlor-6-phenyl-pteridin und 2,6-Dimethyl-morpholin analog Beispiel 1.
Ausbeute: 76 % der Theorie,
Schmelzpunkt: 105 - 115 ° C (Umfällung aus 0,1 N Salzsäure).

Beispiel 9

4-[N-(2-Hydroxy-2-methyl-n-propyl)-ethanolamino]-2,7-bis(3,5-dimethyl-piperidino)-6-phenyl-pteridin

Hergestellt aus 4-[N-(2-Hydroxy-2-methyl-n-propyl)-ethanolamino]-2,7-dichlor-6-phenyl-pteridin und 3,5-Dimethyl-piperidin analog Beispiel 1.
Ausbeute: 50 % der Theorie,
Schmelzpunkt: 206-209 °C

Beispiel 10

4-(N-Ethanol-benzylamino)-2,7-bis(2,6-dimethyl-morpholino)-6-phenyl-pteridin

Hergestellt aus 4-(N-Ethanol-benzylamino)-2,7-dichlor-6-phenyl-pteridin und 2,6-Dimethyl-morpholin analog Beispiel 1.
Ausbeute: 34 % der Theorie,
Schmelzpunkt: 200-202 °C

Beispiel 11

4-[N-(2-Hydroxy-2-methyl-n-propyl)-ethanolamino]-2,7-bis(cis-2,6-dimethyl-morpholino)-6-phenyl-pteridin

Hergestellt aus 4-[N-(2-Hydroxy-2-methyl-n-propyl)-ethanolamino]-2,7-dichlor-6-phenyl-pteridin und cis-2,6-Dimethyl-morpholin analog Beispiel 1.
Ausbeute: 60 % der Theorie,
Schmelzpunkt: 206-209 °C (Isopropanol)

Beispiel 12

4-[N-(2-Hydroxy-2-methyl-n-propyl)-ethanolamino]-2-(2,6-dimethyl-morpholino)-7-morpholino-6-phenyl-pteridin

a) 4-[N-(2-Hydroxy-2-methyl-n-propyl)-ethanolamino]-2-(2,6-dimethyl-morpholino)-7-chlor-6-phenyl-pteridin

2,0 g (5 mMol) 4-[N-(2-Hydroxy-2-methyl-n-propyl)-ethanolamino]-2,7-dichlor-6-phenyl-pteridin werden in 30 ml Dioxan gelöst und mit 1,25 ml (10 mMol) 2,6-Dimethyl-morpholin 2 Stunden bei Raumtemperatur gerührt. Dann wird die Lösung auf Wasser gegeben und der erstarrte Niederschlag abgesaugt, in Methylenchlorid gelöst, getrocknet und einrotiert. Der Rückstand wird über eine Kieselgelsäule mit Chloroform/Methanol = 95:5 chromatographiert.
Ausbeute: 1,1 g (45 % der Theorie),
Schmelzpunkt: 125 °C

b) 4-[N-(2-Hydroxy-2-methyl-n-propyl)-ethanolamino]-2-(2,6-dimethyl-morpholino)-7-morpholino-6-phenyl-pteridin

Hergestellt aus 4-[N-(2-Hydroxy-2-methyl-n-propyl)-ethanolamino]-2-(2,6-dimethyl-morpholino)-7-chlor-6-phenyl-pteridin und Morpholin analog Beispiel 1.
Ausbeute: 93 % der Theorie,
Schmelzpunkt: 125 °C

Beispiel 13

4-[N-(2-Hydroxy-2-methyl-n-propyl)-ethanolamino]-7-(2,6-dimethyl-morpholino)-2-morpholino-6-phenyl-pteridin

a) 4-[N-(2-Hydroxy-2-methyl-n-propyl)-ethanolamino]-2-morpholino-7-chlor-6-phenyl-pteridin

Hergestellt analog Beispiel 12a) aus 4-[N-(2-Hydroxy-2-methyl-n-propyl)-ethanolamino]-2,7-dichlor-6-phenyl-pteridin und Morpholin.
Ausbeute: 64 % der Theorie,
Schmelzpunkt: 152-155 °C

b) 4-[N-(2-Hydroxy-2-methyl-n-propyl)-ethanolamino]-7-(2,6-dimethyl-morpholino)-2-morpholino-6-phenyl-pteridin

Hergestellt aus 4-[N-(2-Hydroxy-2-methyl-n-propyl)-ethanolamino]-2-morpholino-7-chlor-6-phenyl-pteridin und 2,6-Dimethyl-morpholin analog Beispiel 1.
Ausbeute: 54 % der Theorie,
Schmelzpunkt: Sintern bei 125 °C

Beispiel 14

4-(N-Ethyl-benzylamino)-2,7-bis(2,6-dimethyl-morpholino)-6-phenyl-pteridin

Hergestellt aus 4-(N-Ethyl-benzylamino)-2,7-dichlor-6-phenyl-pteridinund 2,6-Dimethyl-morpholin analog Beispiel 1.
Ausbeute: 63 % der Theorie,
Schmelzpunkt: 96 °C

Beispiel I

Dragées mit 75 mg 4-[N-(2-Hydroxy-2-methyl-n-propyl)-ethanolamino]-2,7-bis(2,6-dimethyl-morpholino)-6-phenyl-pteridin

| 1 Dragéekern enthält: | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht:<br>Stempel: | 230 mg<br>9 mm, gewölbt |

Die so hergestellten Drageekerne werden mit einem Film überzogen, der im wesentlichen aus Hydroxypropylmethylcellulose besteht. Die fertigen Filmdragees werden mit Bienenwachs geglänzt.

| | |
|---|---|
| Dragéegewicht: | 245 mg. |

Beispiel II

Tabletten mit 100 mg 4-[N-(2-Hydroxy-2-methyl-n-propyl)-ethanolamino]-2,7-bis(2,6-dimethyl-morpholino)-6-phenyl-pteridin

Zusammensetzung:

| 1 Tablette enthält: | |
|---|---|
| Wirkstoff | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 220,0 mg |

Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50 °C wird erneut gesiebt (1,5 mm-Maschenweite) und das Schmiermittel zugemischt. Die preßfertige Mischung wird zu Tabletten verarbeitet.

| | |
|---|---|
| Tablettengewicht:<br>Durchmesser: | 220 mg<br>10 mm, biplan mit beidseitiger Facette und einseitiger Teilkerbe. |

Beispiel III

Tabletten mit 150 mg 4-[N-(2-Hydroxy-2-methyl-n-propyl)-ethanolamino]-2,7-bis(2,6-dimethyl-morpholino)-6-phenyl-pteridin

Zusammensetzung:

| 1 Tablette enthält: | |
|---|---|
| Wirksubstanz | 150,0 mg |
| Milchzucker pulv. | 89,0 mg |
| Maisstärke | 40,0 mg |
| Kolloide Kieselgelsäure | 10,0 mg |
| Polyvinylpyrrolidon | 10,0 mg |
| Magnesiumstearat | 1,0 mg |
| | 300,0 mg |

Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen. Das bei 45 °C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

Beispiel IV

Hartgelatine-Kapseln mit 150 mg 4-[N-(2-Hydroxy-2-methyl-n-propyl)-ethanolamino]-2,7-bis(2,6-dimethyl-morpholino)-6-phenyl-pteridin

| 1 Kapsel enthält: | |
|---|---|
| Wirkstoff | 150,0 mg |
| Maisstärke getr. | ca. 180,0 mg |
| Milchzucker pulv. | ca. 87,0 mg |
| Magnesiumstearat | 3,0 mg |
| | ca. 420,0 mg |

Herstellung:

Der Wirkstoff wird mit den Hilfsstoffen vermengt, durch ein Sieb von 0,75 mm-Maschenweite gegeben und in einem geeigneten Gerät homogen gemischt.
Die Endmischung wird in Hartgelatine-Kapseln der Größe 1 abgefüllt.

| | |
|---|---|
| Kapselfüllung: | ca. 320 mg |
| Kapselhülle: | Hartgelatine-Kapsel Größe 1. |

Beispiel V

Suppositorien mit 150 mg 4-[N-(2-Hydroxy-2-methyl-n-propyl)-ethanolamino]-2,7-bis(2,6-dimethyl-morpholino)-6-phenyl-pteridin

| 1 Zäpfchen enthält: | |
|---|---|
| Wirkstoff | 150,0 mg |
| Polyäthylenglykol 1500 | 550,0 mg |
| Polyäthylenglykol 6000 | 460,0 mg |
| Polyoxyäthylensorbitanmonostearat | 840,0 mg |
| | 2 000,0 mg |

Herstellung:

Nach dem Aufschmelzen der Suppositorienmasse wird der Wirkstoff darin homogen verteilt und die Schmelze in vorgekühlte Formen gegossen.

Beispiel VI

Suspension mit 50 mg 4-[N-(2-Hydroxy-2-methyl-n-propyl)-ethanolamino]-2,7-bis(2,6-dimethyl-morpholino)-6-phenyl-pteridin

| 100 ml Suspension enthalten: | |
|---|---|
| Wirkstoff | 1,0 g |
| Carboxymethylcellulose-Na-Salz | 0,1 g |
| p-Hydroxybenzoesäuremethylester | 0,05 g |
| p-Hydroxybenzoesäurepropylester | 0,01 g |
| Rohrzucker | 10,0 g |
| Glycerin | 5,0 g |
| Sorbitlösung 70%ig | 20,0 g |
| Aroma | 0,3 g |
| Wasser dest. | ad 100 ml |

Herstellung:

Dest. Wasser wird auf 70 °C erhitzt. Hierin wird unter Rühren p-Hydroxybenzoesäuremethylester und -propylester wobei Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

5 ml Suspension enthalten 50 mg Wirkstoff.

Beispiel VII

Ampullen mit 10 mg 4-[N-(2-Hydroxy-2-methyl-n-propyl)-ethanolamino]-2,7-bis(cis-2,6-dimethyl-morpholino)-6-phenyl-pteridin

| Zusammensetzung: | |
|---|---|
| Wirkstoff<br>0,01 n Salzsäure s.q. Aqua bidest | 10,0 mg<br>ad 2,0 ml |

Herstellung:

Die Wirksubstanz wird in der erforderlichen Menge 0,01 n HCl gelöst, mit Kochsalz isotonisch gestellt, sterilfiltriert und in 2 ml Ampullen abgefüllt. Die Sterilisation erfolgt durch 20 minütiges Erhitzen auf 121 °C.

Beispiel VIII

Ampullen mit 50 mg 4-[N-(2-Hydroxy-2-methyl-n-propyl)-ethanolamino]-2,7-bis(2,6-dimethyl-morpholino)-6-phenyl-pteridin

| Zusammensetzung: | |
|---|---|
| Wirkstoff<br>0,01 n Salzsäure s.q. Aqua bidest | 50,0 mg<br>ad 10,0 ml |

Herstellung:

Die Wirksubstanz wird in der erforderlichen Menge 0,01 n HCl gelöst, mit Kochsalz isotonisch gestellt, sterilfiltriert und in 10 ml Ampullen abgefüllt. Die Sterilisation erfolgt durch 20 minütiges Erhitzen auf 121 °C.

Beispiel IX

Trockenampullen mit 10 mg Doxorubicin und 10 mg 4-[N-(2-Hydroxy-2-methyl-n-propyl)-ethanolamino]-2,7-bis(2,6-dimethyl-morpholino)-6-phenyl-pteridin

Zusammensetzung der Trockenampulle:

| | |
|---|---|
| Doxorubicin<br>Wirksubstanz | 10,0 mg<br>10,0 mg |

Herstellung:

Die beiden Wirksubstanzen werden in der erforderlichen Menge 0,01 n HCl gelöst, sterilfiltriert und lyophylisiert.

Die Lösungsmittelampulle enthält 5 ml Kochsalzlösung.

Vor der Anwendung wird das Lyophylisat in der sterilen physiologischen Kochsalzlösung gelöst.

Beispiel X

Trockenampullen mit 50 mg Doxorubicin und 50 mg 4-[N-(2-Hydroxy-2-methyl-n-propyl)-ethanolamino]-2,7-bis(2,6-dimethyl-morpholino)-6-phenyl-pteridin

Zusammensetzung der Trockenampulle:

| | |
|---|---|
| Doxorubicin | 50,0 mg |
| Wirksubstanz | 50,0 mg |

Herstellung:

Die beiden Wirksubstanzen werden in der erforderlichen Menge 0,01 n HCl gelöst, sterilfiltriert und lyophylisiert.

Die Lösungsmittelampulle enthält 25 ml Kochsalzlösung.

Vor der Anwendung wird das Lyophylisat in der sterilen physiologischen Kochsalzlösung gelöst.

Selbstverständlich können alle übrigen Verbindungen der allgemeinen Formel I als Wirkstoffe in den vorstehenden galenischen Zubereitungen eingesetzt werden.

**Patentansprüche**

**1.** Arzneimittel, enthaltend ein Pteridin der Formel

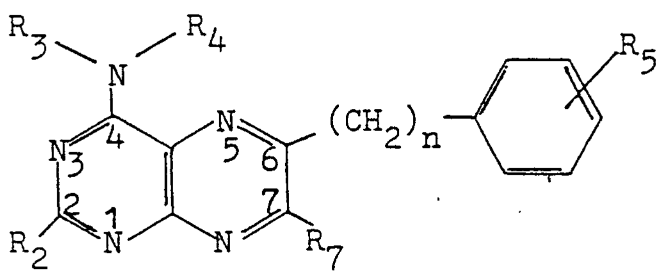

, (I)

in der

n die Zahl 0 oder 1,

$R_3$ eine geradkettige oder verzweigte Alkylgruppe mit 2 bis 6 Kohlenstoffatomen, in welcher mit Ausnahme des zum Stickstoffatom benachbarten Kohlenstoffatoms ein oder zwei Kohlenstoffatome durch eine Hydroxygruppe substituiert sind, und

$R_4$ eine Benzylgruppe oder eine Alkylgruppe mit 1 bis 4 Kohlenstoffatomen, in welcher mit Ausnahme des zum Stickstoffatom benachbarten Kohlenstoffatoms ein Kohlenstoffatom durch eine Hydroxygruppe substituiert sein kann, oder

$R_3$ eine Alkylgruppe mit 1 bis 3 Kohlenstoffatomen und

$R_4$ eine Phenylalkylgruppe mit 1 bis 3 Kohlenstoffatomen,

$R_2$ und $R_7$, die gleich oder verschieden sein können, jeweils eine gegebenenfalls durch eine oder zwei Methyl- oder Ethylgruppen substituierte Pyrrolidino-, Piperidino- oder Morpholinogruppe, und

$R_5$ ein Wasserstoffatom oder eine Methylgruppe bedeuten, eines seiner geometrischen oder optischen Isomeren sowie dessen physiologisch verträglichen Säureadditionssalze mit einer anorganischen oder organischen Säure und mindestens ein Chemotherapeuticum zur Behandlung neoplastischer Erkrankungen aus der Reihe der Naturprodukte.

**2.** Arzneimittel gemäß Anspruch 1, dadurch gekennzeichnet, daß als Chemotherapeuticum Vinblastin, Vincristin, Vindesin, Etoposid, Teniposid, Dactinomycin, Daunorubicin, Doxorubicin, Bleomycin, Mithramycin oder Mitomycin verwendet wird.

**3.** Verfahren zur Herstellung eines Arzneimittels gemäß Anspruch 1, dadurch gekennzeichnet, daß ein Pteridin der Formel I gemäß Anspruch 1 oder dessen physiologisch verträgliches Säureadditionssalz zusammen mit einem Chemotherapeuticum zur Behandlung neoplastischer Erkrankungen aus der Reihe der Naturprodukte in einen oder mehrere inerte übliche Träger eingearbeitet wird.

**4.** Verwendung der Pteridine der Formel I gemäß Anspruch 1 oder deren physiologisch verträgliche Säureadditionssalze zur Herstellung eines Arzneimittels, welches zur Verhinderung der primären und sekundären Resistenz bei der Chemotherapie mit einem Chemotherapeuticum zur Behandlung neoplastischer Erkrankungen aus der Reihe der Naturprodukte geeignet ist.

**5.** Folgende Pteridine der allgemeinen Formel I gemäß Anspruch 1:
4-[N-(2-Hydroxy-2-methyl-n-propyl)-ethanolamino]-2,7-bis(2,6-dimethyl-morpholino)-6-phenyl-pteridin,
4-(N-Ethyl-ethanolamino)-2,7-bis(2,6-dimethyl-morpholino)-6-phenyl-pteridin,
4-(Ethanol-isopropanolamino)-2,7-bis(2,6-dimethyl-morpholino)-6-phenyl-pteridin,
4-[N-(2-Hydroxy-2-methyl-n-propyl)-ethanolamino]-2,7-bis(2-methyl-morpholino)-6-phenyl-pteridin,
4-(Ethanol-isopropanolamino)-2,7-bis(2-methyl-morpholino)-6-benzyl-pteridin,
4-(Ethanol-isopropanolamino)-2,7-bis(2-methyl-morpholino)-6-(o-tolyl)-pteridin,
4-[Bis(2-hydroxy-2-methyl-n-propyl)-amino]-2,7-bis(2,6-dimethyl-morpholino)-6-phenyl-pteridin,
4-(2,3-Dihydroxy-n-propyl-ethanolamino)-2,7-bis(2,6-dimethyl-morpholino)-6-phenyl-pteridin,
4-[N-(2-Hydroxy-2-methyl-n-propyl)-ethanolamino]-2,7-bis(3,5-dimethyl-piperidino)-6-phenyl-pteridin,
4-(N-Ethanol-benzylamino)-2,7-bis(2,6-dimethyl-morpholino)-6-phenyl-pteridin,
4-[N-(2-Hydroxy-2-methyl-n-propyl)-ethanolamino]-2,7-bis(cis-2,6-dimethyl-morpholino)-6-phenyl-pteridin,
4-[N-(2-Hydroxy-2-methyl-n-propyl)-ethanolamino]-2-(2,6-dimethyl-morpholino)-7-morpholino-6-phenyl-pteridin,
4-[N-(2-Hydroxy-2-methyl-n-propyl)-ethanolamino]-7-(2,6-dimethyl-morpholino)-2-morpholino-6-phenyl-pteridin,
4-(N-Ethyl-benzylamino)-2,7-bis(2,6-dimethyl-morpholino)-6-phenyl-pteridin
und deren Säureadditionssalze.

**6.** Pteridine der allgemeinen Formel I gemäß Anspruch 1, in der
n die Zahl 0,
$R_5$ ein Wasserstoffatom,
$R_2$ und $R_7$ jeweils eine 2,6-Dimethyl-morpholinogruppe oder
einer der Reste $R_2$ oder $R_7$ eine Morpholinogruppe und
der andere der Reste $R_2$ oder $R_7$ eine 2,6-Dimethyl-morpholinogruppe,
$R_3$ und $R_4$ zusammen eine N-(2-Hydroxy-2-methyl-n-propyl)-ethanolaminogruppe bedeuten,
deren optische und geometrische Isomere und deren Säureadditionssalze.

**7.** 4-[N-(2-Hydroxy-2-methyl-n-propyl)-ethanolamino]-2,7-bis(2,6-dimethyl-morpholino)-6-phenyl-pteridin, dessen optische und geometrische Isomere und dessen Säureadditionssalze.

**8.** Physiologisch verträgliche Säureadditionssalze der Verbindungen nach mindestens einem der Ansprüche 5 bis 7.

**9.** Verfahren zur Herstellung der Verbindungen nach mindestens einem der Ansprüche 5 bis 8, dadurch gekennzeichnet, daß ein Pteridin der Formel

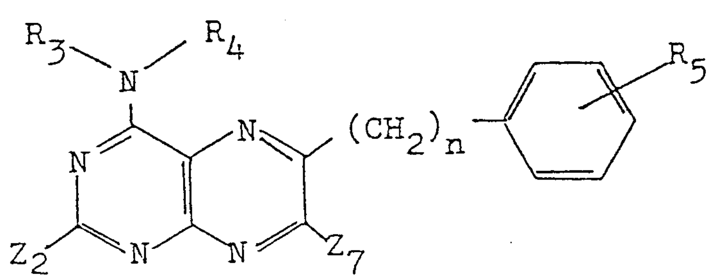

, (II)

in der
$R_3$ bis $R_5$ und n wie in den Ansprüchen 5 bis 7 definiert sind,
einer der Reste $Z_2$ oder $Z_7$ eine nukleophil austauschbare Gruppe darstellt und
der andere der Reste $Z_2$ oder $Z_7$ die für $R_2$ oder $R_7$
in den Ansprüchen 5 bis 7 erwähnten Bedeutungen besitzt oder ebenfalls eine nukleophil austauschbare Guppe darstellt, mit einem Amin der allgemeinen Formel

H - X ,  (III)

in der
X die für $R_2$ oder $R_7$ in den Ansprüchen 5 bis 7 erwähnten Bedeutungen besitzt, umgesetzt wird und
eine so erhaltene Verbindung anschließend gewünschtenfalls in ihre optische und geometrische Isomere aufgetrennt wird und/oder
eine so erhaltene Verbindung in ihr Säureadditionssalz, insbesondere in ihr physiologisch verträgliches Säureadditionssalz, übergeführt wird.

**10.** Arzneimittel zur Verhinderung der primären und sekundären Resistenz von Tumoren bei der Chemotherapie mit einem Chemotherapeutikum zur Behandlung neoplastischer Erkrankungen aus der Reihe der Naturprodukte, enthaltend ein Pteridin nach mindestens einem der Ansprüche 5 bis 7 oder ein physiologisch verträgliches Säureadditionssalz gemäß Anspruch 8 neben einem oder mehreren inerten Trägerstoffen und/oder Verdünnungsmitteln.

**11.** Verfahren zur Herstellung eines Arzneimittels gemäß Anspruch 10, dadurch gekennzeichnet, daß ein Pteridin nach mindestens einem der Ansprüche 5 bis 7 oder ein physiologisch verträgliches Säureadditionssalz gemäß Anspruch 8 in einen oder mehreren inerten Trägerstoffen und/oder Verdünnungsmitteln eingearbeitet wird.

**12.** Verwendung eines Pteridins nach mindestens einem der Ansprüche 5 bis 7 oder ein physiologisch verträgliches Säureadditionssalz gemäß Anspruch 8 zur Herstellung eines Arzneimittels, welches zur Verhinderung der primären und sekundären Resistenz von Tumoren bei der Chemotherapie mit einem Chemotherapeutikum zur Behandlung neoplastischer Erkrankungen aus der Reihe der Naturprodukte geeignet ist.

## Claims

**1.** Pharmaceutical compositions containing a pteridine of formula I

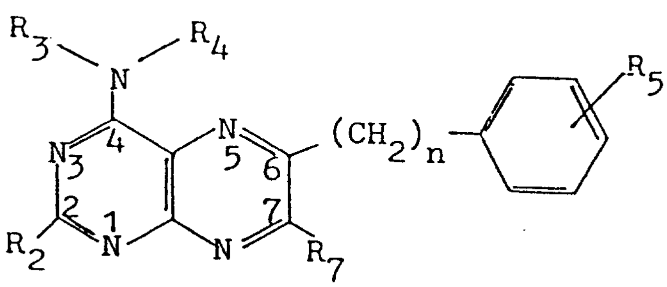

(I)

(wherein
n represents the number 0 or 1,
$R_3$ represents a straight-chained or branched $C_{2-6}$ alkylgroup in which one or two carbon atoms are replaced by a hydroxy group, with the exception of the carbon atom adjacent to the nitrogen atom, and
$R_4$ represents a benzyl group or a $C_{1-4}$ alkyl group in which a carbon atom may be substituted by a hydroxy group, with the exception of the carbon atom adjacent to the nitrogen atom, or
$R_3$ represents a $C_{1-3}$ alkyl group and
$R_4$ represents a phenylalkyl group having 1 to 3 carbon atoms in the alkyl moiety,
$R_2$ and $R_7$, which may be identical or different, each represent a pyrrolidino, piperidino or morpholino group optionally substituted by one or two methyl or ethyl groups, and

$R_5$ represents a hydrogen atom or a methyl group),
one of the geometric or optical isomers thereof and the physiologically acceptable acid addition salts thereof with an inorganic or organic acid and at least one chemotherapeutic agent for the treatment of neoplastic diseases selected from the range of natural products.

**2.** Pharmaceutical composition according to claim 1, characterised in that the chemotherapeutic agent used is vinblastine, vincristine, vindesine, etoposide, teniposide, dactinomycin, daunorubicin, doxorubicin, bleomycin, mithramycin or mitomycin.

**3.** Process for preparing a pharmaceutical composition according to claim 1, characterised in that a pteridine of formula I according to claim 1 or a physiologically acceptable acid addition salt thereof together with a chemotherapeutic agent for the treatment of neoplastic diseases selected from the range of natural products is incorporated in one or more inert conventional carriers.

**4.** Use of the pteridines of formula I according to claim 1 or the physiologically acceptable acid addition salts thereof for preparing a pharmaceutical composition which is suitable for preventing primary and secondary resistance in chemotherapy with a chemotherapeutic agent for the treatment of neoplastic diseases selected from the range of natural products.

**5.** The following pteridines of general formula I according to claim 1:
4-[N-(2-hydroxy-2-methyl-n-propyl)-ethanolamino]-2,7-bis(2,6-dimethyl-morpholino)-6-phenyl-pteridine,
4-(N-ethyl-ethanolamino)-2,7-bis(2,6-dimethyl-morpholino)-6-phenyl-pteridine,
4-(ethanol-isopropanolamino)-2,7-bis(2,6-dimethyl-morpholino)-6-phenyl-pteridine,
4-[N-(2-hydroxy-2-methyl-n-propyl)-ethanolamino]-2,7-bis(2-methyl-morpholino)-6-phenyl-pteridine,
4-(ethanol-isopropylamino)-2,7-bis(2-methyl-morpholino)-6-benzyl-pteridine,
4-(ethanol-isopropanolamino)-2,7-bis(2-methyl-morpholino)-6-(o-tolyl)-pteridine,
4-[bis(2-hydroxy-2-methyl-n-propyl)-amino]-2,7-bis(2,6-dimethyl-morpholino)-6-phenyl-pteridine,
4-(2,3-dihydroxy-n-propyl-ethanolamino)-2,7-bis(2,6-dimethyl-morpholino)-6-phenyl-pteridine,
4-[N-(2-hydroxy-2-methyl-n-propyl)-ethanolamino]-2,7-bis(3,5-dimethyl-piperidino)-6-phenyl-pteridine,
4-(N-ethanol-benzylamino)-2,7-bis(2,6-dimethyl-morpholino)-6-phenyl-pteridine,
4-[N-(2-hydroxy-2-methyl-n-propyl)-ethanolamino]-2,7-bis(cis-2,6-dimethyl-morpholino)-6-phenyl-pteridine,
4-[N-(2-hydroxy-2-methyl-n-propyl)-ethanolamino]-2-(2,6-dimethyl-morpholino)-7-morpholino-6-phenyl-pteridine,
4-[N-(2-hydroxy-2-methyl-n-propyl)-ethanolamino]-7-(2,6-dimethyl-morpholino)-2-morpholino-6-phenyl-pteridine,
4-(N-ethyl-benzylamino)-2,7-bis(2,6-dimethyl-morpholino)-6-phenyl-pteridine
and the acid addition salts thereof.

**6.** Pteridines of formula I according to claim 1, (wherein
n represents the number 0,
$R_5$ represents a hydrogen atom,
$R_2$ and $R_7$ each represent a 2,6-dimethyl-morpholino group or
one of the groups $R_2$ or $R_7$ represents a morpholino group and
the other group $R_2$ or $R_7$ represents a 2,6-dimethyl-morpholino group,
$R_3$ and $R_4$ together represent an N-(2-hydroxy-2-methyl-n-propyl)-ethanolamino group),
the optical and geometric isomers and acid addition salts thereof.

**7.** 4-[N-(2-hydroxy-2-methyl-n-propyl)-ethanolamino]-2,7-bis(2,6-dimethyl-morpholino)-6-phenyl-pteridine, the optical and geometric isomers and acid addition salts thereof.

**8.** Physiologically acceptable acid addition salts of the compounds according to at least one of claims 5 to 7.

**9.** Process for preparing the compounds according to at least one of claims 5 to 8, characterised in that a pteridine of formula

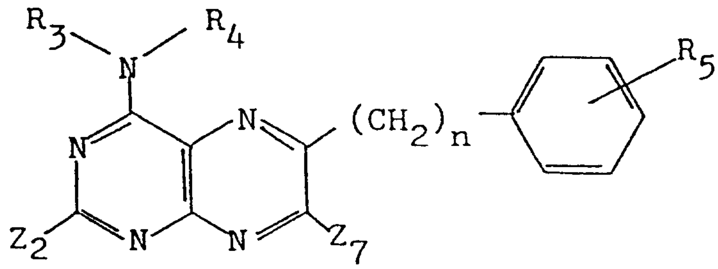

(II)

(wherein
$R_3$ to $R_5$ and n are defined as in claims 5 to 7,
one of the groups $Z_2$ or $Z_7$ represents a nucleophilically exchangeable group and
the other group $Z_2$ or $Z_7$ has the meanings given for $R_2$ or $R_7$ in claims 5 to 7 or also represents a nucleophilically exchangeable group),
is reacted with an amine of general formula III

H - X (III)

(wherein
X has the meanings given for $R_2$ or $R_7$ in claims 5 to 7) and
a compound thus obtained is subsequently, if desired, resolved into the optical and geometric isomers thereof, and/or
a compound thus obtained is converted into an acid addition salt thereof, more particularly a physiologically acceptable acid addition salt thereof.

**10.** Pharmaceutical compositions for preventing primary and secondary resistance of tumours in chemotherapy using a chemotherapeutic agent for treating neoplastic diseases selected from the range of natural products, containing a pteridine according to at least one of claims 5 to 7 or a physiologically acceptable acid addition salt according to claim 8 together with one or more inert carriers and/or diluents.

**11.** Process for preparing a pharmaceutical composition according to claim 10, characterised in that a pteridine according to at least one of claims 5 to 7 or a physiologically acceptable acid addition salt according to claim 8 is incorporated in one or more inert carriers and/or diluents.

**12.** Use of a pteridine according to at least one of claims 5 to 7 or a physiologically acceptable acid addition salt according to claim 8 for preparing a pharmaceutical composition which is suitable for preventing primary and secondary resistance of tumours in chemotherapy using a chemotherapeutic agent for treating neoplastic diseases selected from the range of natural products.

**Revendications**

**1.** Médicament contenant une ptéridine de formule

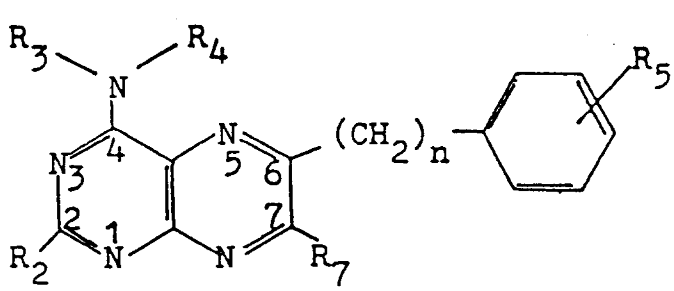

(I)

dans laquelle
n représente le nombre 0 ou 1,

$R_3$ représente un groupe alkyle linéaire ou ramifié de 2 à 6 atomes de carbone dans lequel, à l'exception de l'atome de carbone voisin de l'atome d'azote, un ou deux atomes de carbone sont substitués par un groupe hydroxyle, et
$R_4$ représente un groupe benzyle ou un groupe alkyle de 1 à 4 atomes de carbone dans lequel, à l'exception de l'atome de carbone voisin de l'atome d'azote, un atome de carbone peut être substitué par un groupe hydroxyle, ou
$R_3$ représente un groupe alkyle de 1 à 3 atomes de carbone et $R_4$ représente un groupe phénylalkyle de 1 à 3 atomes de carbone,
$R_2$ et $R_7$, qui peuvent être identiques ou différents, représentent chacun un groupe pyrrolidino, pipéridino ou morpholino éventuellement substitué par un ou deux groupes méthyle ou éthyle, et
$R_5$ représente un atome d'hydrogène ou un groupe méthyle,
l'un de ses isomères géométriques ou optiques ainsi que ses sels d'addition d'acide physiologiquement acceptables avec un acide inorganique ou organique et au moins un agent chimiothérapeutique de la série des produits naturels pour le traitement des maladies néoplasiques.

**2.** Médicament selon la revendication 1, caractérisé en ce que l'on utilise comme agent chimiothérapeutique la vinblastine, la vincristine, la vindésine, l'étoposide, le téniposide, la dactinomycine, la daunorubicine, la doxorubicine, la bléomycine, la mithramycine ou la mitomycine.

**3.** Procédé de préparation d'un médicament selon la revendication 1, caractérisé en ce qu'une ptéridine de formule I selon la revendication 1, ou son sel d'addition d'acide physiologiquement acceptable, est incorporée dans un ou plusieurs véhicules inertes courants en même temps qu'un agent chimiothérapeutique de la série des produits naturels pour le traitement des maladies néoplasiques.

**4.** Utilisation des ptéridines de formule I selon la revendication 1 ou de leurs sels d'addition d'acide physiologiquement acceptables pour la préparation d'un médicament qui convient pour la prévention de la résistance primaire et secondaire lors de la chimiothérapie avec un agent chimiothérapeutique de la série des produits naturels pour le traitement des maladies néoplasiques.

**5.** Ptéridines suivantes de formule générale I selon la revendication 1:
4-[N-(2-hydroxy-2-méthyl-n-propyl)-éthanolamino]-2,7-bis(2,6-diméthyl-morpholino)-6-phényl-ptéridine,
4-[N-éthyl-éthanolamino]-2,7-bis(2,6-diméthyl-morpholino)-6-phényl-ptéridine,
4-(éthanol-isopropanolamino)-2,7-bis(2,6-diméthyl-morpholino)-6-phényl-ptéridine,
4-[N-(2-hydroxy-2-méthyl-n-propyl)-éthanolamino]-2,7-bis(2-méthyl-morpholino)-6-phényl-ptéridine,
4-(éthanol-isopropanolamino)-2,7-bis(2-méthyl-morpholino)-6-benzyl-ptéridine,
4-(éthanol-isopropanolamino)-2,7-bis(2-méthyl-morpholino)-6-(o-tolyl)-ptéridine,
4-[bis(2-hydroxy-2-méthyl-n-propyl)-amino]-2,7-bis(2,6-diméthyl-moroholino)-6-phényl-ptéridine,
4-(2,3-dihydroxy-n-propyl-éthanolamino)-2,7-bis(2,6-diméthyl-morpholino)-6-phényl-ptéridine,
4-[N-(2-hydroxy-2-méthyl-n-propyl)-éthanolamino]-2,7-bis(3,5-diméthyl-pipéridino)-6-phényl-ptéridine,
4-(N-éthanol-benzylamino]-2,7-bis(2,6-diméthyl-morpholino)-6-phényl-ptéridine,
4-[N-(2-hydroxy-2-méthyl-n-propyl)-éthanolamino]-2,7-bis(cis-2,6-diméthyl-morpholino)-6-phényl-ptéridine,
4-[N-(2-hydroxy-2-méthyl-n-propyl)-éthanolamino]-2-(2,6-diméthyl-morpholino)-7-morpholino-6-phényl-ptéridine,
4-[N-(2-hydroxy-2-méthyl-n-propyl)-éthanolamino]-7-(2,6-diméthyl-morpholino)-2-morpholino-6-phényl-ptéridine,
4-(N-éthyl-benzylamino)-2,7-bis(2,6-diméthyl-morpholino)-6-phényl-ptéridine,
et leurs sels d'addition d'acide.

**6.** Ptéridines de formule générale I selon la revendication 1, dans laquelle
n représente le noire 0,
$R_5$ représente un atome d'hydrogène,
$R_2$ et $R_7$ représentent chacun un groupe 2,6-diméthyl-morpholino ou l'un des restes $R_2$ ou $R_7$ représente un groupe morpholino et l'autre des restes $R_2$ ou $R_7$ représente un groupe 2,6-diméthyl-morpholino,
$R_3$ et $R_4$ représentent ensemole un groupe N-(2-hydroxy-2-méthyl-n-propyl)-éthanolamino,
leurs isomères optiques et géométriques et leurs sels d'addition d'acide.

**7.** 4-[N-(2-hydroxy-2-méthyl-n-propyl)-éthanolamino]-2,7-bis(2,6-diméthyl-morpholino)-6-phényl-ptéridine, ses isomères optiques et géométriques et ses sels d'addition d'acide.

**8.** Sels d'addition d'acide physiologiquement acceptables des composés selon au moins l'une des revendications 5 à 7.

**9.** Procédé de préparation des composés selon au moins l'une des revendications 5 à 8, caractérisé en ce qu'une ptéridine de formule

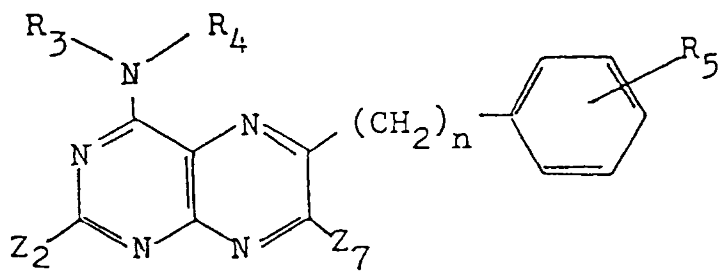

(II)

dans laquelle
$R_3$ à $R_5$ et n sont définis comme dans les revendications 5 à 7,
l'un des restes $Z_2$ ou $Z_7$ représente un groupe susceptible d'échange nucléophile et
l'autre des restes $Z_2$ ou $Z_7$ possède les significations indiquées pour $R_2$ ou $R_7$ dans les revendications 5 à 7 ou représente aussi un groupe susceptible d'échange nucléophile, est mise à réagir avec une amine de formule générale

H - X (III)

dans laquelle
X possède les significations indiquées pour $R_2$ ou $R_7$ dans les revendications 5 à 7, et
un composé ainsi obtenu est ensuite résolu si on le souhaite en ses isomères optiques et géométriques, et/ou
un composé ainsi obtenu est converti en son sel d'addition d'acide, en particulier en son sel d'addition d'acide physiologiquement acceptable.

**10.** Médicament pour la prévention de la résistance primaire et secondaire des tumeurs lors de la chimiothérapie avec un agent chimiothérapeutique de la série des produits naturels pour le traitement des maladies néoplasiques, contenant une ptéridine selon au moins l'une des revendications 5 à 7 ou un sel d'addition d'acide physiologiquement acceptable selon la revendications 8 ainsi qu'un ou plusieurs véhicules et/ou diluants inertes.

**11.** Procédé de préparation d'un médicament selon la revendication 10, caractérisé en ce qu'une ptéridine selon au moins l'une des revendications 5 à 7, ou un sel d'addition d'acide physiologiquement acceptable selon la revendication 8, est incorporée dans un ou plusieurs véhicules et/ou diluants inertes.

**12.** Utilisation d'une ptéridine selon au moins l'une des revendications 5 à 7 ou d'un sel d'addition d'acide physiologiquement acceptable selon la revendication 8 pour la préparation d'un médicament qui convient pour la prévention de la résistance primaire et secondaire des tumeurs lors de la chimiothérapie avec un agent chimiothérapeutique de la série des produits naturels pour le traitement des maladies néoplasiques.